# EUROPÄISCHE PATENTANMELDUNG

(11) **EP 1 431 268 A1**
(43) Veröffentlichungstag der Anmeldung: **23.06.2004**
(21) Anmeldenummer: 03028912.8
(22) Anmeldetag: 17.12.2003
(51) Int. Cl.: C07C 67/05, C07C 69/16

(54) **Verfahren zur Gewinnung von 9,10-Dihydroxyanthracencarboxylestern**

(30) Priorität: 21.12.2002 DE 10260550
(71) Anmelder: RÜTGERS Chemicals AG, 44579 Castrop-Rauxel (DE)
(72) Erfinder: Polaczek, Jerzy, Dr.Inz., 02-784 Warszawa (PL); Domanowski, Wojciech, Mgr.Inz., 03-226 Warszawa (PL); Pielichowski, Jan, Prof.Dr.Hab.Inz., 30-076 Krakow (PL); Machowska, Zofia, Mgr.Inz., 01-912 Warszawa (PL); Fuhrmann, Edgar, Dr., 44579 Castrop-Rauxel (DE); Talbiersky, Joerg, Dr., 46282 Dorsten (DE)
(74) Vertreter: COHAUSZ & FLORACK

(57) **Zusammenfassung**

In einem Verfahren zur Herstellung von 9,10-Dihydroxyanthracencarbonsäureester und dessen Abkömmlingen durch katalytische Oxidation von Anthracen wird Anthracen in der flüssigen Phase in einem Carbonsäuremedium in Gegenwart eines organischen Metallsalzes und eines Aktivierungsmittels für das Metallsalz bei einer Temperatur von etwa 40 °C und mehr unter Einwirkung von Sauerstoff und Licht behandelt, der Niederschlag abgetrennt, der flüssige Rest mit Veresterungsmitteln behandelt und der erhaltene Ester aus dem Reaktionsgemisch isoliert.

## Beschreibung

Die Erfindung betrifft ein Verfahren zur Oxidation von Anthracen in der Flüssigphase, in dem als Hauptprodukt ein 9,10-Dihydroxyanthracencarboxylester anfällt.

Die Oxidation von Anthracen zu Anthrachinon gehört seit langem zum Stand der Technik und ist unter anderem in H.G. Franck, J.W. Stadelhofer, Industrielle Aromatenchemie, Springer Verlag 1987, beschrieben. Im allgemeinen wird dabei Anthrachinon durch katalytische Gasphasenoxidation aus Anthracen in Gegenwart eines molekularen Sauerstoff enthaltenden Gases hergestellt, wobei Schwermetalloxide als Katalysatoren eingesetzt werden. Die Herstellung von Anthrachinon mittels Gasphasenoxidation belastet durch freigesetzte Anthracen- und Anthrachinondämpfe die Umwelt erheblich. Nachteilig ist ferner, dass die Gasphasenoxidation eine niedrige Selektivität aufweist und während der Reaktion unerwünschte Nebenprodukte entstehen. Die Ausbeute an Anthrachinon nach der Gasphasenoxidation liegt je nach Katalysator bei 80 bis 95 %.

Neben der Gasphasenoxidation ist die Flüssigphasenoxidation, beispielsweise mit Dichromat oder Salpetersäure, ausgehend von festem Anthracen bekannt. Die Verwendung von Dichromat oder Salpetersäure in einer Flüssigphasenoxidation ist zur Vermeidung ökologischer Nachteile immer mit einer aufwendigen Wasseraufbereitung verbunden.

In der PL 0 295 761 wird ein Verfahren beschrieben, in dem Wasserstoffperoxid als Oxidationsmittel eingesetzt wird. Nachteilig an dem Verfahren ist jedoch, dass der Verbrauch an Wasserstoffperoxid so hoch ist, dass die Herstellung von Anthrachinon nicht mehr wirtschaftlich ist.

In den letzten Jahren wird am Markt eine Überkapazität von Anthrachinon beobachtet. Verfahren zur Herstellung von Anthrachinon durch Oxidation von Anthracen haben daher etwas an Bedeutung verloren. Dagegen sind 9,10-Dihydroxyanthracen, ein Zwischenprodukt der Oxidation von Anthracen, oder dessen Ester von großem Interesse. Diese Verbindungen eignen sich als Ausgangsmaterial zur Herstellung neuer wertvoller Polymere.

Da die Oxidation von 9,10-Dihydroxyanthracen zu Anthrachinon schnell, dagegen die von Anthracen zu 9,10-Dihydroxyanthracen langsam verläuft, ist eine selektive Umsetzung von Anthracen zu 9,10-Dihydroxyanthracen kaum möglich. Die Herstellung von 9,10-Dihydroxyanthracen ist also nur auf indirekten Wege, beispielsweise durch Reduktion von Anthrachinon mit Wasserstoff, durchführbar. Ein mehrstufiges Verfahren erweist sich aber häufig als unwirtschaftlich, wenn die Kosten für die Oxidation und Reduktion addiert werden.

Der Erfindung liegt die Aufgabe zugrunde, ein Verfahren zur Oxidation von Anthracen zu 9,10-Dihydroxyanthracencarbonsäureester oder dessen Abkömmlingen bereitzustellen, das in der Flüssigphase unter milden Bedingungen zu vergleichsweise hohen Ausbeuten führt.

Gelöst wird diese Aufgabe durch ein Verfahren zur Herstellung von 9,10-Dihydroxyanthracencarbonsäureester und dessen Abkömmlingen durch katalytische Oxidation von Anthracen, in dem man Anthracen in der flüssigen Phase in einem Carbonsäuremedium in Gegenwart eines organischen Metallsalzes und eines Aktivierungsmittels für das Metallsalz bei einer Temperatur von etwa 40 °C und mehr unter Einwirkung von Sauerstoff und Licht behandelt, den Niederschlag abtrennt, den flüssigen Rest mit Veresterungsmitteln behandelt und den erhaltenen Ester aus dem Reaktionsgemisch isoliert.

In diesem Verfahren erfolgt die Oxidation mit den sich bei der Sauerstoffoxidation von Carbonsäuren *in situ* bildenden Peroxysäuren, wobei das Produkt in Form eines Monoesters oder Diesters der Carbonsäure anfällt.

Nach diesem Verfahren kann ein festes Anthracen und ein katalytisches System in einem Carbonsäuremedium suspendiert und dann unter Sauerstoffzufuhr und Lichtzutritt Anthracen zu einem 9,10-Dihydroxyanthracen-Carbonsäureester oxidiert werden.

9,10-Dihydroxyanthracen ist sehr instabil und wird in Gegenwart von Luft spontan zu Anthrachinon oxidiert. Eine Unterbrechung der Reaktion auf der Dihydroxyanthracen-Zwischenstufe konnte deshalb nicht erwartet werden. Auch der Einsatz von Schutzgruppen wie Acetoxygruppen war nicht erfolgreich. In der Vergangenheit wurde 9,10-Dihydroxyanthracen daher ausschließlich durch Reduktion erzeugt. Wenn auch etwa noch 20% Anthrachinon erhalten werden, so ist der Erfolg des erfindungsgemäßen Verfahrens dennoch überraschend.

Als Ausgangsmaterial kann ein übliches technisches Anthracen eingesetzt werden, wie es beispielsweise von der Rütgers Chemicals AG bereitgestellt wird. Dieses weist eine Reinheit von etwa 96% auf und enthält als Hauptverunreinigungen Phenanthren (2 Gew.%), Carbazol (< 1 Gew.%) und Fluoren (< 1 Gew.%).

Das Carbonsäuremedium kann gebildet werden durch Ameisensäure, Essigsäure, Eisessig, Propionsäure, Isopropionsäure, deren Gemische, weitere niedere organische Mono- oder Dicarbonsäuren und deren Säureanhydride. Geeignete Dicarbonsäuren sind bespielsweise Adipinsäure, Maleinsäure und Phthalsäure. Insbesondere geeignet ist ein Gemisch aus Säure und Säureanhydrid. Die Säuren können gegebenfalls in organischen Lösungsmitteln vorliegen. Gemäß einer besonders bevorzugten Ausführungsform ist das Carbonsäuremedium ein Gemisch aus Eisessig und Essigsäureanhydrid. Das Carbonsäuremedium soll möglichst wasserfrei sein. Ein Wassergehalt von bis zu etwa 1% im Carbonsäuremedium ist unschädlich.

Das erfindungsgemäß eingesetzte katalytische System enthält ein aktiviertes organisches Metallsalz. Geeignete organische Metallsalze sind die Metallsalze der oben genannten, das Carbonsäuremedium bildenden Carbonsäuren. Zur Metallsalzbildung besonders geignete Metalle sind Übergangsmetalle in höherer Oxidationsstufe, +III, IV, V, VI und VII. Bevorzugte Salze sind solche von Mangan, Kobalt, Molybdän, Nickel, Zirkonium und Hafnium in den zuvorgenannten Oxidationszahlen. Besonders bevorzugt sind Mangan und Kobalt.

Bevorzugte organische Metallsalze sind beispielsweise Mangan- und Kobaltacetat oder Mangancarbonat.

Die Metallsalze werden vorzugsweise in Konzentrationen von 0,0005 bis 0,2 mol/l, vorzugsweise 0,002 bis 0,05 mol/l eingesetzt.

Die Aktivierung des Metallsalzes erfolgt durch ein geeignetes Aktivierungsmittel. Aktivierungsmittel sind beispielsweise Bromide, Jodide oder Chloride. Besonders bevorzugt sind Bromide. Die Aktvierungsmittel in Form der zuvor genannten Halogenide können als Metallsalz oder organisches Halogenid eingesetzt werden. Geeignete Metallhalogenide sind beispielsweise die Alkalimetall- und Erdalkalimetallbromide und Ammoniumbromid. Geeignete organische Bromide sind beispielsweise Trimethyl-*tert*.-butylammoniumbromid, Methintribromid (Bromoform), Acetylentetrabromid und Phenylbromid. Besonders bevorzugt als Aktivierungsmittel für das Metallsalz sind Natriumbromid und Ammoniumbromid

Das Aktivierungsmittel kann im Reaktionsgemisch in einer Konzentration von 0,001 bis 0,5 mol/l, vorzugsweise 0,002 bis 0,1 mol/l, enthalten sein.

Bevorzugt werden das aktivierte organische Metallsalz oder ein Gemisch aus Metallsalzen und das Aktivierungsmittel in einem Gewichtsverhältnis von 0,8:1 bis 1:0,8, vorzugsweise etwa 1:1 eingesetzt.

Das erfindungsgemäße Verfahren kann in einem üblichen Rührreaktor batchweise oder kontinuierlich durchgeführt werden.

Nach dem erfindungsgemäßen Verfahren werden Anthracen und das katalytische System in dem Carbonsäuremedium suspendiert. Dies kann bei Raumtemperatur erfolgen. Unter Zufuhr von Sauerstoff und unter Lichtzutritt kann das erfindungsgemäße Verfahren in einem Temperaturbereich von vorzugsweise 40 bis 100 °C, vorzugsweise 55 bis 85 °C, durchgeführt werden. Sauerstoff wird vorzugsweise gasförmig, besonders bevorzugt als im wesentlichen reiner Sauerstoff, zugeführt. Die Reaktionsdauer kann 3 bis 24 Stunden betragen.

Der sich während der Reaktion bildende Niederschlag kann von der Reaktionslauge getrennt werden. Dieser Niederschlag umfaßt beispielsweise nach einer 14stündigen Reaktion bei 60 °C in einem Essigsäure/Essigsäureanhydrid-medium 70 bis 72 Gew.% 9-Acetoxy-10-hydroxyanthracen als Hauptprodukt der Oxidation. Außerdem werden Anthrachinon (20 Gew.%), Phenanthren (2 Gew.%), 9-Anthron (4 Gew.%) sowie kleinere Mengen (unter 1 Gew.%) von nicht umgesetztem Anthracen im Niederschlag nachgewiesen.

Das Abtrennen des Niederschlags kann durch Sedimentation, Zentrifugieren und/oder Filtration erfolgen. Das Abtrennen des Niederschlags geschieht vorzugsweise bei Raumtemperatur.

Das im Niederschlag enthaltene Rohanthrachinon kann aus einer der verwendeten Carbonsäuren, beispielsweise Essigsäure, umkristallisiert werden. Es liegt dann mit einer Reinheit von über 99,2 Gew.% vor. Die Ausbeute von Anthrachinon, bezogen auf Anthracen, beträgt etwa 20 %.

Der flüssige Rest nach Abtrennung des Niederschlags wird dann entweder unter Vakuum eingeengt oder mit einem Fällungsmittel, beispielsweise Wasser, behandelt. Um das 9-Carboxy-10-hydroxyanthracen in einen oxidationsfesten 9,10-Dihydroxyanthracencarbonsäureester zu überführen, kann das angereicherte Zwischenprodukt zusätzlich mit einem Carbonsäureanhydrid, beispielsweise Essigsäureanhydrid, behandelt werden. Diese Behandlung kann in Gegenwart von Basen wie Pyridin oder Natriumacetat erfolgen. Dadurch wird die vollständige Umsetzung von 9-Carboxy-10-hydroxy-anthracen zu 9,10-Diacetcarboxyanthracen erreicht.

Um Restmengen von Anthrachinon aus dem Produkt zu entfernen, kann dem füssigen Rückstand nach Abtrennen des Niederschlags ein Metall wie Zink oder Aluminium, vorzugsweise als Pulver, zugesetzt werden. Wird als Carbonsäuremedium ein Gemisch aus Essigsäure/Essigsäureanhydrid eingesetzt, wird das Produkt durch Kristallisation aus dem Reaktionsgemisch in Form von gelben Nadeln mit einem Schmelzpunkt von 272 °C isoliert. Die Reinheit des Produkts ist höher als 99,5%. Die Ausbeute von 9,10-Diacetoxyanthracen, bezogen auf Anthracen, beträgt 67 %.

Erfindungsgemäß erhältliche 9,10-Dihydroxyanthracencarbonsäureester sind 9,10-Dialkylcarboxyloxyanthracene, 9,10-Alkylarylcarboxyloxyanthracene und 9,10-Diarylcarboxyloxyanthracene, wobei Alkyl eine Kohlenwasserstoffgruppe mit 1 bis 8 Kohlenstoffatomen und Aryl einen substituierten oder nicht substituierten aromatischen Kohlenwasserstoffrest mit 5 bis 14 Kohlenstoffatomen bedeuten kann.

Das folgende Beispiel dient der weiteren Erläuterung der Erfindung.

### Beispiel

In einen mit magnetischem Rührer, Thermometer, Sauerstoffzufuhr und Rückflusskühler versehenen Dreihalsrundkolben eines Fassungsvermögens von 150 ml werden 20 ml Essigsäure (rein), 30 ml Essigsäureanhydrid, 2,5 g technisches Anthracen, 0,08 g Mangancarbonat (rein) und 0,08 g Natriumbromid eingeführt. Das Gemisch wird unter Rühren und kontinuierlicher Begasung mit Sauerstoff (10 ml/min) auf 60 °C erwärmt und 24 Stunden lang oxidiert. Das Reaktionsgemisch wird jeweils nach 10 und 14stündiger Reaktion auf seine chemische Zusammensetzung gaschromatographisch untersucht. Die Ergebnisse dieser Untersuchung sind in der Tabelle gezeigt.

Die Reaktion wird als beendet angesehen, wenn der Anteil des nicht umgesetzten Anthracens kleiner als 1 Gew.% ist. Nach Abkühlen des Reaktionsgemischs auf Raumtemperatur werden 0,8 g Rohanthrachinon abfiltriert und aus 30 ml Essigsäure umkristallisiert. Das Anthrachinon hat eine Reinheit von 99,6 %.

Das unter Vakuum (40 kPa) mit Wasserstrahlpumpe eingeengte Filtrat wird durch Zugabe von 5 ml Essigsäureanhydrid, 7g Pyridin und 2 g Zinkpulver 30 min lang bei Raumtemperatur behandelt. Als Reaktionsprodukt wird festes 9,10-Diacetoxyanthracen abgetrennt und aus Essigsäure umkristallisiert. Das Produkt wird in Form von gelben Nadeln mit einem Schmelzpunkt von 272 °C isoliert. Die Ausbeute ist 77 % der Theorie, Gesamtausbeute an Anthrachinon und 9,10-Diacetoxyanthracen überschreiten 95 % der theoretisch erzielbaren Ausbeute.

Die in der folgenden Tabelle zusammengestellten Ergebnisse zeigen die Produktzusammensetzung nach 10 und 14stündiger Reaktion unter Verwendung eines Essigsäure/Essigsäureanhydrids als Carbonsäuremedium.

| Zeit[h] | Zusammensetzung des Produkts, Gew.% | | | | | |
|---|---|---|---|---|---|---|
| | Anthracen | Anthrachinon | Anthron | 9-Hydroxy-10-acetoxyanthracen | 9-Acetoxyanthracen | Phenanthren |
| 0 | 96 | 0 | 0 | 0 | 0 | 2 |
| 10 | 75 | 2 | 1,2 | 12 | 2 | 2 |
| 14 | < 1 | 20 | 4 | 72 | Spuren | 2 |

## Patentansprüche

1. Verfahren zur Herstellung von 9,10-Dihydroxyanthracencarbonsäureester und dessen Abkömmlingen durch katalytische Oxidation von Anthracen, **dadurch gekennzeichnet, dass** man Anthracen in der flüssigen Phase in einem Carbonsäuremedium in Gegenwart eines organischen Metallsalzes und eines Aktivierungsmittels für das Metallsalz bei einer Temperatur von etwa 40 °C und mehr unter Einwirkung von Sauerstoff und Licht behandelt, den Niederschlag abtrennt, den flüssigen Rest mit Veresterungsmitteln behandelt und den erhaltenen Ester aus dem Reaktionsgemisch isoliert.

2. Verfahren nach Anspruch 1, **dadurch gekennzeichnet, dass** das man als Carbonsäuremedium ein Gemisch aus Essigsäure und Essigsäureanhydrid einsetzt.

3. Verfahren nach Anspruch 1 oder 2, **dadurch gekennzeichnet, dass** man als katalytisches System ein aktiviertes organisches Metallsalz oder ein Gemisch von Metallsalzen und einem Aktivierungsmittel einsetzt.

4. Verfahren nach einem der Ansprüche 1 bis 3, **dadurch gekennzeichnet, daß** man Mangan- oder Kobaltacetat oder deren Gemische als organisches Metallsalz einsetzt.

5. Verfahren nach einem der Ansprüche 1 bis 4, **dadurch gekennzeichnet, daß** man ein anorganisches Bromid wie Natriumbromid oder Ammoniumbromid oder ein organisches Bromid wie Trietyl-*tert.*-butylammoniumbromid als Aktivierungsmittel einsetzt.

6. Verfahren nach einem der Ansprüche 1 bis 5, **dadurch gekennzeichnet, dass** man den flüssigen Rest einengt.

7. Verfahren nach Anspruch 6, **dadurch gekennzeichnet, dass** man das eingeengte Medium mit einem Carbonsäureanhydrid als Veresterungsmittel und gegebenenfalls einer Base behandelt.

8. Verfahren nach einem der Ansprüche 1 bis 7, **dadurch gekennzeichnet, dass** man den abgetrennten Niederschlag aus einer Carbonsäure umkristallisiert.
